Europäisches Patentamt

European Patent Office

Office européen des brevets

(11) Publication number: **0 060 717**
A1

# (12) EUROPEAN PATENT APPLICATION

(21) Application number: 82301331.3

(51) Int. Cl.³: **C 07 C 69/14**, C 07 C 67/08

(22) Date of filing: 16.03.82

(30) Priority: 17.03.81 GB 8108335

(43) Date of publication of application: 22.09.82
Bulletin 82/38

(84) Designated Contracting States: BE DE FR GB IT NL

(71) Applicant: BP Chemicals Limited, Belgrave House 76 Buckingham Palace Road, London, SW1W 0SU (GB)

(72) Inventor: Yeomans, Bertram, 9 Mill Road Swanland, Hull Humberside (GB)

(74) Representative: Harry, John et al, BP INTERNATIONAL LIMITED Patents and Licensing Division Chertsey Road, Sunbury-on-Thames Middlesex TW16 7LN (GB)

(54) Process for the production of methyl acetate by esterifying methanol with acetic acid.

(57) Methyl acetate is produced by esterifying methanol with acetic acid in the presence of an esterification catalyst and separating the products in the presence of an entrainer using two distillation columns. Suitable entrainers include toluene, diisobutylether, n- and iso-butyl acetate and methyl isobutyl ketone.

EP 0 060 717 A1

0060717

1

## PROCESS FOR THE PRODUCTION OF METHYL ACETATE BY ESTERIFYING METHANOL WITH ACETIC ACID.

The present invention relates to an improved process for the production of methyl acetate by esterifying methanol with acetic acid.

Methyl acetate, besides retaining its usefulness in solvent applications, is gaining importance as an intermediate in the production of other valuable products. Owing to its unfavourable physical properties (viz. composition of azeotropes and solubility in water) pure methyl acetate containing low concentrations of methanol and water is not readily obtainable using conventional processes. A conventional esterification process which may be used for the production of methyl acetate comprises the addition of a feed mixture comprising excess methanol and acetic acid to an esterifier containing aqueous acetic acid and a strong acid catalyst (e.g. toluene-para-sulphonic acid) under reflux conditions. A mixture of methyl acetate, reaction water and the excess methanol is taken overhead as a distillate product. A typical ester distillate product obtained by use of a 2:1 molar ratio feed of methanol:acetic acid should theoretically comprise 59.9% w/w methyl acetate, 26% w/w methanol and 14.5% w/w water. Conventionally purification of such a crude ester distillate is effected in a multi-stage process which typically incorporates hydroselection (for removal of methanol) and azeotropic drying (for removal of water). Additional distillation steps are required to recover methanol from the hydroselection base product and methyl acetate from the decanter water phase/entrainer for recycle.

We have now found that methyl acetate of improved purity in terms of methanol and water content and hence of greater suitability as a feedstock in certain reactions, such as the production of acetic anhydride by carbonylation, can be produced in a simpler manner than hitherto.

Accordingly, the present invention provides a process for the production of methyl acetate which process comprises reacting in an esterification reaction vessel methanol at elevated temperature with acetic acid in the presence of an esterification catalyst to form a product comprising methyl acetate and water, in a first distillation column distilling from the product in the presence of an entrainer which is sparingly soluble in water and which forms a minimum boiling point azeotrope therewith an overhead fraction comprising methyl acetate, entrainer and water, separating water from methyl acetate and entrainer and thereafter in a second distillation column separating methyl acetate as an overhead fraction from entrainer by distillation.

The entrainer may suitably be added to the esterification reaction vessel or to the first distillation column or to both. The process may be operated batchwise or continuously, preferably continuously.

A preferred embodiment of the invention comprises continuously feeding methanol, acetic acid and entrainer to an esterification reaction vessel containing entrainer, acetic acid and esterification catalyst at elevated temperature thereby producing a product comprising entrainer, catalyst, methyl acetate and water, distilling from the product in a first distillation column an overhead fraction comprising methyl acetate, water and entrainer, condensing the overhead fraction, separating at least part of the condensed overhead fraction by decantation into a lower aqueous phase and an upper organic phase containing methyl acetate and entrainer and thereafter separating the organic phase by distillation in a second distillation column into an overhead methyl acetate fraction and a residue entrainer fraction.

Whilst the esterification reaction vessel may be independent from the first distillation column it is preferred that they be integral. Preferably the reaction vessel forms the kettle at the base of the first distillation column. Whenever the reaction vessel is independent from the first distillation column it is preferred to recycle the residue from the base of this column back to the reaction vessel.

Preferably the feed comprises equimolar amounts of methanol and acetic acid.

The elevated temperature may vary over a moderately wide range, but must be sufficient, in the embodiments wherein the reaction vessel is integral with the first distillation column, to distil methyl acetate, water and entrainer out of the reaction mixture. Thus at atmospheric pressure suitable reaction temperatures are in the range 100°C to 120°C, preferably 105° to 115°C. The reaction vessel may be provided with, for example, steam coils, or other forms of heating.

Methyl acetate, water and entrainer are removed as an overhead fraction from the first column by fractional distillation. The reflux necessary for successful fractionation may be provided in two ways. A part of the vapour leaving the head of the column may be condensed for example in a vertical condenser and the condensate returned directly to the column without any further separation step (primary reflux). Alternatively, or in addition, a portion of either the aqueous phase or the organic phase may be returned to the column (secondary reflux).

In the embodiment in which the reaction vessel constitutes the kettle of the first distillation column the reflux ratio is preferably sufficient to maintain the water content of the reaction vessel at not more than 10% by weight of the reactor contents and preferably not more than 3% by weight. It is also desirable that the loss of acetic acid overhead in the first column should be kept to a minimum. The preferred primary reflux ratios used to provide the control on acetic acid loss overhead whilst effecting the necessary separation may suitably be in the range 1:2 to 10:1, preferably 1:1 to 5:1.

A secondary reflux of either organic- or water-phase from the decanter may be used to control the water level in the system at the correct level. Preferably a secondary reflux of decanted organic phase is used to control the water content and the reflux ratio may suitably be in the range 2:1 to 1:10, preferably, 1:1 to 1:5.

The mixture of methyl acetate and entrainer are separated in a second column from which the methyl acetate is recovered overhead and the entrainer is recovered as a residue fraction.

Using butyl acetate as the entrainer, the second column may suitably comprise from 5 to 25, preferably from 10 to 15 theoretical plates. The kettle temperature may be in the range 55 to 75°C, preferably from 60 to 70°C, the heads temperature may be in the range 50 to 60°C, preferably from 55 to 58°C and the column may suitably be operated at a reflux ratio in the range 2:1 to 1:10, preferably from 1:1 to 1:5. The temperatures refer to atmospheric pressure operation and for sub- or superatmospheric operation should be adjusted accordingly. Using entrainers other than butyl acetate, the aforesaid ranges may require adjustment depending upon the nature of the entrainer but such adjustment should be well within the competence of a person ordinarily skilled in the art.

A particularly preferred embodiment of the invention comprises continuously feeding methanol, acetic acid and entrainer to a reaction kettle surmounted by a first distillation column, which kettle contains entrainer, acetic acid, water and esterification catalyst at elevated temperature thereby producing a product comprising methyl acetate, water, unreacted methanol, unreacted acetic acid, catalyst and entrainer, distilling from the product an overhead fraction comprising principally methyl acetate, water and entrainer together with some methanol and acetic acid, leaving in the kettle a residue fraction comprising catalyst, acetic acid, entrainer and some water, condensing the overhead fraction, returning a part of the condesate to the column as primary reflux, separating the remainder of the condensate by decantation into an oil phase comprising principally methyl acetate and entrainer together with some water, methanol and acetic acid and an aqueous phase, returning a portion of the oil phase to the column as secondary reflux, feeding the remainder of the oil phase to a second distillation column wherein there is separated overhead a fraction principally comprising methyl acetate, but containing also water and methanol and trace amounts of acetic acid and entrainer, from a residue fraction principally comprising entrainer together with methyl acetate and trace amounts of methanol, acetic acid and water, and recycling the residue fraction from the second distillation column to the kettle of the first distillation column.

The methyl acetate recovered can be further purified if so desired by hydroselection (for removal of methanol) and azeotropic drying (for removal of water) and/or by selective adsorption using a molecular sieve and/or by reaction with acetic anhydride.

With respect to the reactants, it is immaterial whether the methanol and acetic acid contain water.

The entrainer may be any hydrocarbon, ether, ester or ketone which is sparingly soluble in water and which forms a minimum boiling point azeotrope with water. Examples of suitable entrainers are toluene, diisobutyl ether, normal- and iso-butyl acetate and methyl isobutyl ketone. Preferably the entrainer is an acetic acid ester such as butyl acetate. An advantage of using an acetic acid ester is that it can be formed 'in situ', for example n-butyl acetate can be formed by incorporating n-butanol in the reaction mixture. Methyl acetate (boiling point 57°C) and n-butyl acetate (boiling point 126°C) can be readily separated by distillation.

The amount of entrainer present may suitably be greater than 10%, preferably from 20 to 60%, by weight based on the total weight of the reaction mixture.

The esterification catalyst may suitably be a mineral acid such as sulphuric acid or an organic acid such as toluene-para-sulphonic acid, of which toluene-para-sulphonic acid is preferred. The esterification catalyst may be present in an amount from 0.1 to 10% by weight of the reaction mixture and preferably from 2 to 6% w/w.

The methyl acetate recovered from the second distillation column is suitable, with or without further purification, as feed to a process for producing acetic anhydride and/or acetic acid by reaction with carbon monoxide in the presence of a metallic carbonylation catalyst, eg a Group VIII noble metal, of which rhodium is preferred, and a promoter comprising a halogen in free or combined form, eg iodine or methyl iodide or acetyl iodide, and optionally in the presence also of co-promoters, such as alkyl or aryl phosphines and organo-nitrogen compounds. Suitable catalysts, promoters, co-promoters and reaction conditions are described in for example UK Patents Nos. 1468940, 1523346 and 1538783 and European Patent No. 8396.

The invention will now be illustrated by reference to the following Example.

Example

A feed mixture (2728g) comprising 49% w/w acetic acid, 27% w/w methanol and 24% w/w n-butyl acetate was fed over 6 hours into a reaction still kettle (containing 820g of a refluxing mixture comprising 33.7% w/w acetic acid, 61% w/w n-butyl acetate, 1.4% w/w water and 3.9% w/w toluene-para-sulphonic acid), fitted with a 25-plate Oldershaw commmn and overhead condensation/decantation system. The overhead two-phase product obtained using a primary reflux ratio of 3:1 and a secondary reflux ratio of 1:3 was separated to give an oil phase (2340g) comprising 67.8% w/w methyl acetate, 27.3% w/w n-butyl acetate 1.1% w/w methanol, 0.02% w/w acetic acid and 3.8% w/w water, and an aqueous phase (357g). The oil phase (2340g) obtained by separation of the overhead two-phase product was charged to a 3-litre flask fitted with a 10 plate Oldershaw column and overhead condensation/decantation system. The overhead product (1687g) obtained using a reflux ratio of 8:1 comprised 93.2% methyl acetate, 0.05% n-butyl acetate, 1.5% methanol, 0.002% acetic acid and 5.2% water. The balance (ca. 650g) principally comprising butyl acetate obtained as a distillation residue was recycled with the esterification feed to the reaction still.

Comparison Test

A feed mixture (4122g) comprising 48.4% w/w acetic acid and 51.6% w/w methanol (equivalent to a molar ratio of 1:2 respectively) was fed over 6.5. hours into an esterifier (ca. 2 1 capacity) containing a refluxing mixture (800g) of 50% w/w acetic acid, 48% w/w water and 2% w/w toluene-para-sulphonic acid and fitted with a 5-plate Oldershaw column. The methyl acetate product (4123g), which comprised 57.5% w/w methyl acetate, 27.9% w/w methanol, 13.8% w/w water and 0.8% w/w acetic acid, was taken overhead using a reflux ratio of 3:1 and a stillhead temperature of ca. 70°C/1 bar.

This is not an example of the present invention because no entrainer was employed.

The ester product obtained overhead from the first column in the Example was contaminated with far less water, methanol and acetic acid than the product obtained by the conventional process employed in the Comparison Test.

    0060717

Claims:

1. A process for the production of methyl acetate which process comprises reacting in an esterification reaction vessel methanol at elevated temperature with acetic acid in the presence of an esterification catalyst to form a product comprising methyl acetate and water, in a first distillation column distilling from the product in the presence of an entrainer which is sparingly soluble in water and which forms a minimum boiling point azeotrope therewith an overhead fraction comprising methyl acetate, entrainer and water, separating water from methyl acetate and entrainer and thereafter in a second distillation column separating methyl acetate as an overhead fraction from entrainer by distillation.

2. A process according to claim 1 wherein methanol, acetic acid and entrainer are continuously fed to an esterification reaction vessel containing entrainer, acetic acid and esterification catalyst at elevated temperature thereby producing a product comprising entrainer, methyl acetate and water, distilling from the product in a first distillation column an overhead fraction comprising methyl acetate, water and entrainer, condensing the overhead fraction, separating at least part of the overhead fraction by decantation into a lower aqueous phase and an upper organic phase containing methyl acetate and entrainer and thereafter separating the organic phase by distillation in a second distillation column into an overhead methyl acetate fraction and a residue entrainer fraction.

3. A process according to either claim 1 or claim 2 wherein the reaction vessel forms the kettle at the base of the first distillation column.

4. A process according to any one of the preceding claims wherein the reaction vessel is integral with the first distillation column and the esterification reaction temperature is in the range 110 to 120°C.

5. A process according to any one of claims 2 to 4 wherein the reflux to the first distillation column is provided by condensing a part of the vapour leaving the head of the column and returning the condensate directly to the column without any further separation step (primary reflux) and/or by returning to the column a portion of either the separated aqueous phase or organic phase (secondary reflux).

6. A process according to claim 5 wherein the primary reflux ratio is in the range 1:2 to 10:1.

7. A process according to claim 5 wherein the secondary reflux is provided by returning to the column a part of the organic phase and the reflux ratio is in the range 2:1 to 1:10.

8. A process according to any one of the preceding claims wherein the entrainer is selected from toluene, diisobutylether, normal- and iso-butyl acetate and methyl isobutyl ketone.

9. A process according to any one of the preceding claims wherein the entrainer is n-butyl acetate.

10. A process according to claim 9 wherein the n-butylacetate is formed 'in situ' by incorporating n-butanol in the esterification reaction mixture.

11. A process for the production of methyl acetate which process comprises continuously feeding methanol, acetic acid and entrainer to a reaction kettle surmounted by a first distillation column, which kettle contains entrainer, acetic acid, water and esterification catalyst at elevated temperature thereby producing a product comprising methyl acetate, water, unreacted methanol, unreacted acetic acid, catalyst and entrainer, distilling from the product an overhead fraction comprising principally methyl acetate, water and entrainer together with some methanol and acetic acid, leaving in the kettle a residue fraction comprising catalyst, acetic acid, entrainer and some water, condensing the overhead fraction, returning a part of the condensate to the column as primary reflux, separating the remainder of the condensate by decantation into an oil phase comprising principally methyl acetate and entrainer together with some water, methanol and acetic acid and an aqueous phase, returning a portion of the oil phase to the column as secondary reflux, feeding the remainder of the oil phase to a second distillation column wherein there is separated overhead a fraction prinicpally comprising methyl acetate, but containing also water and methanol and trace amounts of acetic acid and entrainer, from a residue fraction principally comprising entrainer together with methyl acetate and trace amounts of methanol, acetic acid and water, and recycling the residue fraction from the second distillation column to the kettle of the first distillation.

0060717

European Patent
Office

**EUROPEAN SEARCH REPORT**

Application number

EP  82 30 1331

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | FR-A-1 194 137  (S.A.TURBINIA) <br><br> *Page  2, table; pages 3-4, example 2; page 5, claims; figure 1* | 1-3,5, 8,11 | C 07 C  69/14 <br> C 07 C  67/08 |
| X | US-A-2 787 636  (L.ALHERTIERE) <br><br> *Column 1, lines 39-72; column 2, lines  1-24;  columns 5-6; claims 1-6* | 1-3,5, 8,9,11 | |

TECHNICAL FIELDS
SEARCHED (Int. Cl. ³)

C 07 C  69/00
C 07 C  67/00

The present search report has been drawn up for all claims

| Place of search | Date of completion of the search | Examiner |
|---|---|---|
| THE HAGUE | 04-06-1982 | KINZINGER J.M. |